# EUROPEAN PATENT APPLICATION

(11) **EP 1 106 703 A1**
(43) Date of publication of application: **13.06.2001**
(21) Application number: 99204219.2
(22) Date of filing: 09.12.1999
(51) Int. Cl.: C12Q 1/70, A61K 39/42, C07K 14/15

(54) **Testing xenografts and sources thereof for retrovirus**

(71) Applicant: Amsterdam Support Diagnostics B.V., 1105 BA Amsterdam (NL)
(72) Inventor: Mang, Rui, 1104 LM Amsterdam (NL); Van der Kuyl, Antoinette Cornelia, 1231 VS Loosdrecht (NL)
(74) Representative: Ottevangers, Sietse Ulbe

(57) **Abstract**

The invention relates to the field of xenotransplantation and/or to the detection of retrovirus. The invention provides an isolated or recombinant nucleic acid comprising a consensus retroviral sequence allowing detection of a retroviral genome by nucleic acid hybridisation and/or amplification, said consensus sequence at least partly derived from a novel domestic pig retrovirus genome.

### Annex to the application documents - subsequently filed sequences listing

SEQUENCE LISTING

<110> Amsterdam Support Diagnostics B.V.

<120> Testing xenografts and sources thereof for retrovirus

<130> P51976EP00

<140> EP 99204219.2
<141> 1999-12-09

<160> 30

<170> PatentIn Ver. 2.1

<210> 1
<211> 23
<212> DNA
<213> Artificial Sequence

<220>
<223> Description of Artificial Sequence: Upstream
primer BBRT1, based on consensus retroviral RT
gene

<400> 1
ctcggatccg tnytnccnca rgg 23

<210> 2
<211> 21
<212> DNA
<213> Artificial Sequence

<220>
<223> Description of Artificial Sequence: Downstream
primer BBRT2, based on consensus retroviral RT
gene

<400> 2
ctcgtcgacr tcrtccatrt a 21

<210> 3
<211> 20
<212> DNA
<213> Baboon endogenous virus

<220>
<223> /note="Upstream primer L3505, based on the RT
sequence of BaEV, which has a C type pol gene"

<400> 3
tggactcgac ttccccaggg                                20

<210> 4
<211> 29
<212> DNA
<213> Baboon endogenous virus

<220>
<223> /note="Downstream primer H3616, based on the RT
      sequence of BaEV, which has a C type pol gene"

<400> 4
tatagcggcc gcaggaggtc atctacata                      29

<210> 5
<211> 26
<212> DNA
<213> Artificial Sequence

<220>
<223> Description of Artificial Sequence: Upstream
      primer PERV-FP, based on PERV protease gene

<400> 5
gctacaacca ttaggaaaac taaaag                         26

<210> 6
<211> 25
<212> DNA
<213> Artificial Sequence

<220>
<223> Description of Artificial Sequence: Downstream
      primer PERV-RP, based on PERV protease gene

<400> 6
aaccaggact gtatatcttg atcag                          25

<210> 7
<211> 20
<212> DNA
<213> Domestic pig endogenous retrovirus

<220>
<223> /note="Upstream gag-primer PC41, based on DoPEV"

<400> 7

cgatgggacc atacaagagg 20

<210> 8
<211> 18
<212> DNA
<213> Domestic pig endogenous retrovirus

<220>
<223> /note="Downstream gag-primer PC4, based on DoPEV"

<400> 8
gccaagtctg tttgaccc 18

<210> 9
<211> 20
<212> DNA
<213> Domestic pig endogenous retrovirus

<220>
<223> /note="Upstream pol-primer PCRT1, based on DoPEV"

<400> 9
cctaatggat accaggagcc 20

<210> 10
<211> 20
<212> DNA
<213> Domestic pig endogenous retrovirus

<220>
<223> /note="Downstream pol-primer PC10, based on DoPEV"

<400> 10
agtcaagtca ggtctgctgg 20

<210> 11
<211> 18
<212> DNA
<213> Domestic pig endogenous retrovirus

<220>
<223> /note="Upstream primer PC1, based on P1.1 clone of
DoPEV"

<400> 11
gcagtcagat tcttaacc 18

<210> 12
<211> 18
<212> DNA
<213> Domestic pig endogenous retrovirus

<220>
<223> /note="Downstream primer PC2, based on P1.1. clone
of DoPEV"

<400> 12
atagtgggct tggaaccc                                    18


<210> 13
<211> 18
<212> DNA
<213> Domestic pig endogenous retrovirus

<220>
<223> /note="Upstream primer PC3, based on P1.1 clone of
DoPEV"

<400> 13
cgattgttac ccgaggcc                                    18


<210> 14
<211> 18
<212> DNA
<213> Domestic pig endogenous retrovirus

<220>
<223> /note="Upstream primer PC5, based on P1.1 clone of
DoPEV"

<400> 14
tttgacactg aagagcgc                                    18


<210> 15
<211> 18
<212> DNA
<213> Domestic pig endogenous retrovirus

<220>
<223> /note="Downstream primer PC6, based on P1.1. clone
of DoPEV"

<400> 15
ttcagggcct cctaaccg                                    18

<210> 16
<211> 18
<212> DNA
<213> Domestic pig endogenous retrovirus

<220>
<223> /note="Upstream primer PC7, based on P1.1 clone of
DoPEV"

<400> 16
ttgacgatgt cggttagg 18


<210> 17
<211> 18
<212> DNA
<213> Domestic pig endogenous retrovirus

<220>
<223> /note="Downstream primer PC8, based on P1.1. clone
of DoPEV"

<400> 17
ctgcctccat cagtaggg 18


<210> 18
<211> 18
<212> DNA
<213> Domestic pig endogenous retrovirus

<220>
<223> /note="Upstream primer PC9, based on P1.1 clone of
DoPEV"

<400> 18
gacctcttac tagctggc 18


<210> 19
<211> 18
<212> DNA
<213> Domestic pig endogenous retrovirus

<220>
<223> /note="Upstream primer PC11, based on P1.1. clone
of DoPEV"

<400> 19
tggaggtact agatgagg 18

<210> 20
<211> 18
<212> DNA
<213> Domestic pig endogenous retrovirus

<220>
<223> /note="Downstream primer PC12, based on P1.1. clone
of DoPEV"

<400> 20
gtgacttcag tgaagtcc                                    18


<210> 21
<211> 18
<212> DNA
<213> Domestic pig endogenous retrovirus

<220>
<223> /note="Upstream primer PC13, based on P1.1. clone
of DoPEV"

<400> 21
aacactgtgg actgtccc                                    18


<210> 22
<211> 18
<212> DNA
<213> Domestic pig endogenous retrovirus

<220>
<223> /note="Downstream primer PC14, based on P1.1. clone
of DoPEV"

<400> 22
gcagctgatt tctcaagc                                    18


<210> 23
<211> 18
<212> DNA
<213> Domestic pig endogenous retrovirus

<220>
<223> /note="Upstream primer PC15, based on P1.1. clone
of DoPEV"

<400> 23
gaccacacca gaagctgg                                    18

<210> 24
<211> 18
<212> DNA
<213> Domestic pig endogenous retrovirus

<220>
<223> /note="Downstream primer PC16, based on P1.1. clone
of DoPEV"

<400> 24
ggccaatata gtcaaggc 18

<210> 25
<211> 127
<212> DNA
<213> Artificial Sequence

<220>
<223> Description of Artificial Sequence: RT sequence
amplified from pig genome

<400> 25
ggactcgact tccccagggc ttcaaaaact ccccgaccat ctttgacgaa gccctacaca 60
gagacctggc caacttcagg atccaacacc cccaggtgac cctcctccag tatgtagatg 120
acctcct 127

<210> 26
<211> 126
<212> DNA
<213> Artificial Sequence

<220>
<223> Description of Artificial Sequence: RT sequence
amplified from pig genome

<400> 26
ggactcgact tccccagggt tcaaaaactc tcccactctt tttagcggca acttagctgc 60
tgacctggct aaatttcctg gacaaaaact gggctgtgtt ttgctacagt atgtagatga 120
cctcct 126

<210> 27
<211> 117
<212> DNA
<213> Artificial Sequence

<220>
<223> Description of Artificial Sequence: RT sequence
amplified from pig genome

<400> 27
gtgtttcctc agggcatgaa aaacagtcct accatatgcc aacaattagt aggagatgtg 60
ttattaactt tttgaagcaa ataccctact atacaacttt accattacat ggacgac 117

<210> 28
<211> 116
<212> DNA
<213> Artificial Sequence

<220>
<223> Description of Artificial Sequence: RT sequence
amplified from pig genome

<400> 28
gttttgccac agggaatgaa aaacagtcca acattgtgcc aaaaatttgt tgacatgggc 60
ttgatacagg tcaaacaaaa atatccttct atctacctga tccattacat gacgac 116

<210> 29
<211> 117
<212> DNA
<213> Artificial Sequence

<220>
<223> Description of Artificial Sequence: RT sequence
amplified from pig genome

<400> 29
gtcttaccac agggtatgat taatagtccg accttatgcc aagagtttgt ggcgcgggcc 60
ttgactcctg tgagacactt gtttcctcaa tttatcttat atcactacat ggatgac 117

<210> 30
<211> 10753
<212> DNA
<213> Domestic pig endogenous retrovirus

<220>
<223> /note="Nucleic acid sequence of DoPEV genomic
fragment comprising a 11kb sequence obtained from
clone P1.1"

<400> 30
   cagctatgcc catgattacg caagctattt aggtgcacta tagatactca agctatgcat 60
   caagcttggt ccgagctcgg atccactaga acggccgcca gtgtgctgga attcgccttg 120
   ctatcatcac taatgggagt cgactcgatc tagtgtccat ggtacaaggt aaatgactct 180
   tttaaagtga agaataccga aggaaaacag aaaaatctag ctggaagcac taagccaatt 240
   ttgtcattat caaagagtaa agaattactc ccaagaatca aatggtattc ttctccagct 300
   gaactttgag taacttttgt tgttgtggcc acagctgtgg caatgtggga tcctttcacc 360
   cacacccact gcaggattcc atcccgcacc tctgcagcaa ccagagcccc tgcaatacag 420
   tgggaattct gaactttgag tcattttttt aatgcaaaac tcttggttat acaaagcaaa 480
   gtgatatttt gacttccttt tggacctcaa accctatatt gtgcttgcaa aatatttatt 540
   ccatacctct ctttatattt caataaaatt tccttacaga tttcactaga gagaatccat 600
   atgtgcataa tatatttggt atcaagtaat gggattttta gtaaaatcag gaagcacgtt 660
   tgtccacaga ggttcagttt catcaaaaat atggaaagat atataagcta gtttttagca 720
   tgggcttaaa gatattttct tggattgagt ttgcatcttc tattgaattc ttctttgcac 780
   atttctattc atatatacct ttattgttat catttttatg gtttcaaaag atttgaacac 840
   aagagattta aatcacagca gcatttgctc tgttcaaaac tggtgcaaaa ggcagcaaga 900
   tttcttttcc atgttctaca aactagagaa ttactaactg caaataagga aaaaaaatct 960
   ccatttgatg ccaaatacaa attgtggtcc tgattcctgg aaagaggctt caaagtggac 1020
   cttgatgcta tgaaataaaa aggatgcaaa aatagttgac atgaaagctt tctttgtgca 1080
   cacctgtgca agtctttgat attctattga ttcataagtt ctctaaggaa taaatttcag 1140
   cattaaatta ccccaaacag tcatgaagcc tttctttcaa tctcttaaat gctcttgcat 1200
   cagggaattt tattatgtaa tgggaaaata tgaaatatga aaattccctg tctgctgcct 1260
   cctttttttg atctaggcca gggctatctt aacaggcctg acatttaggt cacctgcctg 1320
   taactcaaat ttgacctcat ggtcttattt ctttccactt ttgctgcagt acagcaaggg 1380
   aaaaaaatat gtaatttttt gtttttcctt attcactttt tcttttaatg gtgtctggta 1440
   gaaaggtttt tttaaaatac attgtgatca ttctatttct taaatcatga ccaattggtt 1500
   ttaaaatcct ggttctttgc cttctagggt aaacctgtat gttcctgtat ttttgcctca 1560
   tgtaatttct ctgggacaaa aaatataata agttttcatg ggttgtatag ttagagagac 1620
   ctattcagag agttgctatg cagtttggca aactgcttat ctcagtgggc ctcagtttcc 1680
   taaactgtca aaaggagaga tcataacacc ttcttcatag ctctctaaaa gagaagtgag 1740
   atgacggaaa aacatagtca gtagtgtgct taacgtatcg catattagct atgtgacagg 1800
   aagcaggtta aataacaatc agaagagaaa ttggctaaaa gttcaaacca taacttcaaa 1860
   agtaatgaga gaaacaaagg atgacaaata ccatatttgg tttcatttta cttttacaag 1920
   gaagcttctc tgtgataatt tatatatata tggcaatttg agagcagaat tatctttaga 1980
   agtagaaaga actctaaaga taattctgct ctcaaattgt ccattgtgat gatagccgaa 2040
   aatataatcc atgaagatac aattgatcaa tataggataa ttttaaacac aaatgttaaa 2100
   aaatgtatca tgttcttaca attattatca ttttcaagat cagtcatatg tttccacata 2160
   gaagctacct atattatcca gtaccaaatt ttcatgatca tgcaaaaaaa aaaaaaaaaa 2220
   aaggcagtaa gtgacatttc caaagagcaa aatattataa atgaaaccgt catacccacc 2280
   aacattgaaa caaggctaaa ggctctgctg cctttttttg tttaagtaaa aacggctgca 2340
   tatttcagca ggctacacaa cccccagagg atggcaggaa agacgtgatg ggaaaaacta 2400
   aaatgggatt ttgaacaaaa tgagagaatt tagagccaaa taaagaaccc tagaccaagc 2460
   cagagccttg tttccatata catatgtgtg tgtatatata tatatatatg tcttccccac 2520
   ctcccccaga ccagggattg aatcctagct gcagctgcga cctgtagttg tggcgacaca 2580
   atgccagatc ttttaaccta ctctgctggg ccagggatcg aacccacacc tccacagcaa 2640
   cccaagcttc tgcagtcaga ttcttaaccc attgcaccac aggggaaacc cctacatttt 2700
   ctttttagta atattaattt aatattattt tccaccatgg tctatcccag aagattggat 2760
   atagttccct ggctatacag taggacctta ttgtctatcc attacacatg taataattta 2820
   ttgcaatgat ttttaaaggc acctttacga gattctgagg ttctgccaga caagggaatc 2880
   actggacaaa ggacaaatta tgttctcctg ccaataccaa tcaactctat gatgtggctc 2940
   ccctggcaca cagcagcact gcttaattct agatatgttt tgattagaca cagatgcatt 3000
   taattagcag tttgtatcat caattagaac atgaacccaa aaagtcacct tcctgcttag 3060
   atatgctttg aacacagatt ttagtatgtg atgattcctg aagaataaaa agagagggga 3120
   ttctactgaa atcattaaaa tctgaaaata tctcacctga ggatgtaatc cagaaagcaa 3180
   gaactcttga gtaagctaat agttatagtc ctacacttag ctttagtcag agattctggg 3240
   taccatagaa taaggggaaa tggtgatgat aataatggaa aagctacatt tcatttttat 3300
   gtcattttcc ccaaagtatt ttcaaaagcc acccctagcc aattttacat ggaggaaaga 3360
   cttatgtaaa aggaggttgt actgttgcag attcctgaaa tcttccattt ggtcaaaatt 3420
   tgttcattta attctctgat ttcaggaggg aaaacaacag ctactactgt gactactact 3480
   gaaatattac tccagtactt atttatccaa ttaaaatata tcctatccat caacccacta 3540
   tttgatttta cagtggagga atatgataag aggcgcctga aatatctcct ccgggggagt 3600
   ggtcagatgg acgaagcgat tgttacctga ggccttgtcc tcacagtttg tcttgcgtca 3660
   ccggcacggg gtgggacttc acacaatggg taaatcggct tccaagccca ctatcttaga 3720
   gtatatgttt aagaatttta agaaaggttt tctagagatt atggtgttaa gctgagtcct 3780
   ggaaaactgt gtaccctctg taccctagag tggccctcct ttggtattgg ctggcctccg 3840
   gaagggacct tagatgtgtt cacagttaca gcagtatata atgttataac aggagaccct 3900
   ggacacccag accaatttct gcatattaat caatggttgg aaattgccta tttgaggtcc 3960
   ccatgcgttc ggttctgcac cacagaccag ggacagtgta gggtgctagt agcccagtcc 4020
   aagaagaaaa ccttgtctca aaaaaaaggt gccacctatt tatcaagggg aacctgagga 4080
   ctcaccccct atgccaccac cttatgtgct gctggcccca cctccgggag ccgaaggtcc 4140
   cagataccag atagcccact gccttctgtt tctcctcctc cttctgcttc tgaagttccc 4200
   gaggtgatat cacccctgca gccagattct ccagaaccta ttagcaggca tctccagtca 4260
   gctcagggag cagccactcc agcattacaa atgcccttac aggagacacg gggaccacag 4320
   caagtcgccc ccgatgggac catacaagag gggggacatt tttattatta ccagcccttt 4380
   tccactgctg atctcctcaa ctggaaacac catactccct catattctga aaagcctcag 4440
   gcactgattg atctcttaga attcattttc cagacacact gtcccacctg gatagactgc 4500
   cggcaactcc ttttcactct atttgacact gaagagcgct ggcgaattgt aacagaagcc 4560
   caaaaatggc ttcaggccaa tgcggggggt caaacagact tggcaaactg ggtgagggaa 4620
   gccttcccag aagaaaaccc ccactgggac tatgacattg aagaagggaa atgtaaccta 4680
   gagaggtacc agcaggcctt cctacagggg gcaaaagctg gagctaaaag ccctactaat 4740
   atagctaaga tcagtgagat actgcaagag ccgaatgaaa gcccagccaa attctgtgaa 4800
   agaccatgtg aagctttcca aagctacaca ctttttgatt ccaaggcccc tgagaagcaa 4860
   cggatgataa atgctgcctt tgtgggacag gcccaatcag atatccataa aaagctacag 4920
   aaattagaag gatttgcagg agagaatgcc actaaactgt tggaaatagc caacaagttt 4980
   tttattaacc aagaccaagt ggcccgccgt gatgcagaaa agagaataaa acaaaaagtg 5040
   actcttccgg cagctgccct gtcaaaaccg gtgcccccag tgggaccacc ccgtaaggga 5100
   caaggcacaa ggccagggaa aaggagtccc ctggaacatg accaatgagc ttactgcaaa 5160
   aaaaagggac attggaaaaa tgaatgcccc aaccgtcctg agaaaaagac caaggcccca 5220
   cctagccagt accgatctga gccattcagc cccaacctga tcggcctagc tgagtctgat 5280
   tggggggacc aggctctctc caactgggcc cccgagagcc tatggtcaga attcaagtaa 5340
   ggggccaccc catagacttt atggttgaca caggtgccaa actttctgtg gttacacaac 5400
   ctgtagctcc cctctcgggg aaagaaacca ccattgttgg ggccacaggt aaccaaaccc 5460
   acaggccctt cagcggtcct tgacgatgtc ggttaggagg ccatgaagta atacatgaat 5520
   ttctttacct gcctgactgc ccagtccccc tgatggaaag agaccttctg gctaaaatgg 5580
   gggcacaaat ttccttttct actgatggat cagcccaact caaattgaca gaaccccccc 5640
   ctctccttta atcatggctc ttgctgtaaa aagagaagag aaatggcgct tgtactcttc 5700
   cccttcagaa atggggacca ttcccccaga actagagact aaatacccat tggtttgggc 5760
   taaaggaaac ccaccaggac tggcaaaatg ccatgttcct gtcttgattg atctaaaacc 5820
   cggagttcaa ctggtaaagc tacgacaata ctcaattccc agagaagctt gactgggaat 5880
   ccaggttcac ctggataggc tgcttcagca tggactgtta ataaaatgtc agtccccttg 5940
   gaacacccct ttactgccca tgaaaaaacc ggaaacacag gactaccgcc cagtacagga 6000
   cttgagagcc ataaataaaa aaatagtcac attatacccg tccatcccta acccgtatac 6060
   cctgctaggg ctgatcccct ccactgctga gtggtttact tgcctggact taaaagacac 6120
   ctttttctgc ctccgggtgg cacctgccag tcagcccttg tttgcttttg aatgggaaaa 6180
   tccacataga gggacaaagg aacagctcac ctggacaaga ctcccacaag ggttcaaaaa 6240
   ctcaaccact ctttttagcg gcgccttagc tgctgacctg gctgaatttc ctggacaaaa 6300
   actgggctgt gttttgctac agtatgttga tgacctctta ctagctggca tcaaggaggc 6360
   tcagtgccta gagggaacaa gaattctcct ttccctactg atggaggcag ggtatccggt 6420
   atcaaagaaa aaggcacaaa tctgtaaaaa acaagtcaag tatctgggct tcaatatcat 6480
   gcagggccga cgaatgctgg ggactgaaaa aagcaggcag tttgtgccat tcctgttcct 6540
   accacccgct ggaaggttca tgagttccta ggggcagcag gattctgctg gatatggatc 6600
   cccgggtttt cagacttggt caggcctctg tatgaggctc taaaagggga agaaaaggct 6660
   ccccttaaat ggggaccaag ccaagagaca gtgtttcaga caatcaaaac aaaactcact 6720
   gaggctccag ccttgggact tcctcatata accagagaat ttaacctttt tgttcatgag 6780
   aaaaatggga tggctttggg agtcttaact caagaatttg ggccttggcg gagaccagtg 6840
   gcatatctgt ctaaacaaat tgactcagtt gctgccagat ggcctccctg cctctgagcg 6900
   ctggcagcta ccaccttgtt ggtaagagag gctgacaagt tgaccctggg acaaaacctc 6960
   aatgttaaag tcccacactc agttgtaacc ctaatggata ccaggagcca ccattggctc 7020
   acacatgcac gaatgactca atatcaaggg ttactatgtg aaaatccacg ggtaaacctg 7080
   gaggttgtac gaactctgaa cccagctact tttctgccag atgaagcggg acccccagac 7140
   cataactgcc tggaggtact agatgaggtg ttttccagca gacctgactt gactgataaa 7200
   ccactccaaa atcctgacct ggttttatat actgatgaca acagctttat ggaggatgaa 7260
   aaaaggagga caggatatgc tgtggtatct gacttaaaag tgataaaagc agaggtgctt 7320
   cctcaaggct ggtcagcaca gagagcagaa ttatgggcct taatcagaac actggaactc 7380
   agccgagacc agtgagttaa tatctatacc gactcacgat ttgcctttgc aaccttacat 7440
   gtccatggag ccttatataa agaaagagga ctcctcactg ctaaaggaaa agggattaaa 7500
   aaccaggctg aaatcttaaa gcttctagag acagtatggg aacctaaaga agttgcagtt 7560
   atccactgta aaggccacca aacccagtga caaaaggaaa ccgtagtgct gatgcagctg 7620
   ccagacaggc agcccgggga caacagccag acaaacctaa ggtcttgctg gccccagaag 7680
   tgccatctgt ccccagatat tttccaaagg aagaaaaatg gataagggag gaaggaggaa 7740
   ctaaaattaa aacgggatgg tgggtcacac aggacctccg agtttatgtt ccagaacagc 7800
   tggcctacaa actagtccac caacaacatg agctaaccca catgggcaaa actgccttag 7860
   agaccctact gggctggtac tatctcattg ctcgtcttcc tgcactctgc tcctctgtct 7920
   cccaaagatg cataacctgt ttacagaata atgcccacca ggggccccac agaccagctg 7980
   gggcacaaca ctgtggactg tccccatttg atgatataaa agtggacttc actgaagtca 8040
   cccctaatga agggtacaaa tatttactag tttttatctg tactttttca ggatgggtag 8100
   aggccttccc tacccgaact gaaaaggcaa gggaagtaac taaggcactc ctgagagaca 8160
   ttattcccag atatgaaatg cctctgacca ttgggtcaga taatggactg gcatttgtgg 8220
   ccaaaacagt ataacaggtg gcaaaagctt tacagatctg ataaaaacta cattctgctt 8280
   atcaccctca aagttcaggg aaagtagaac acatgaacca aacccttaaa cagacactgg 8340
   caaagctatg tcaggaaact ggcttgccct ggatagacat gttgctggtg gcccccttaa 8400
   aagtgagatg ctcacctcga gtgggaatag gattctcgcc atttaaaacc ttatatggaa 8460
   ggccacctcc actaatcagt ttaaaaggaa acaccaggga gttgcgagac ttggaattac 8520
   ataaacaatt acaaggactg gggctcacta tttcttggtt atataaatgg gtcacagaca 8580
   gaatacctgt gtccttaggt ataacagtac acccccacaa gcagggggac caggtgtgga 8640
   caaaggactg gaaaaaggag tctttgaggc tggtatggaa gagcccctat actgtaatac 8700
   taaccacccc cactgctctt aaagtaacag gaatagatgc ttggatccat cattatagag 8760
   ttaaaccggc tggcccagct gatgaccgtg gagagtggga gactgccctc gatccagaga 8820
   gacctctttg cctgacaata cagagaagga gacaacaatc tcaaagccct gctctgacca 8880
   caccagaagc tggtcagtca acgcatggct gaagcttgag aaatcagctg cctgtgagcc 8940
   caaatggaag gagggtttca aaataagcat tcgcattgac gaaagaggcc ttgaccctgg 9000
   agctgtacta catttccaga aaatcacgtt tgcgctccaa gctgcctctc acagggtctt 9060
   tcattccttt tatgaagaga taaaaaacac cccacctccc attctggtca caactaagaa 9120
   cttgttctta gccctagcaa aaactattgc acaaactctc aaggtctcct cttgttatgt 9180
   atgcggggga acgaacacag gagaccactg gccttggcaa gccagagaaa tggacccatt 9240
   agagcctcat aatgagactg cataccccca actcactgat ggggtatggc tcctcaaaac 9300
   tgccattata gggaaaaatt gtcttgcgag cggggaggac agtttaatgt ttcagttgaa 9360
   agctaacatg tttaggccta aggtattata atctgactgc ccaaaagact caatggtggt 9420
   catccttcaa tctctcagag ccgaagccta atcccttgtc taacttttcc ctccttcgac 9480
   aagcatggaa agatataata tccagtggca ggcaccaaag ggagtatatt gaatttgtgg 9540
   aaaaatggcc tattccatac tccccccaaa ttgggctggg gcatgtgtac tgggaacaat 9600
   cagaccttcc ttcttcctgc tcccgctgtc actaggggag caactggaaa ttcctatata 9660
   taggcacagg gtgcccagag agaccggagg gctctccaaa ttggagactg gaaagatgat 9720
   gaatggcccc caggatggat aattcaatat tatgggcctg ccacctgggc agaggatggc 9780
   tcacacccct atctatatgc tgaactgcat catcagacta caggcggttt tggaacttat 9840
   aacaaatgac actgcaaggg ccttgactat attggcccaa caacaaacta aaatgaacag 9900
   tacagtctac caaaatcact tggccttgga ttatttactt gcttctgaag gaggggtttg 9960
   tgaaaatttt aacttaagta attgctgtct acaaatagat gacactggaa aggtggtaga 10020
   agaaattgct gaccacaaga ctaaacttgc ccatgtacct gtacaaactt ggaaaggatg 10080
   ggatacagac agtctctttg gtaaatggtt ctcttggttg ggagaaatta agacaatggt 10140
   tgaaataatc atggttatac tcacagggtg cctacttgtt ccctgccttg taactctcct 10200
   gataaatatt ataaaaggtt ttataaaaag catggttaaa aaaaaaaaca gcctcccacc 10260
   tgctacttat cagaggctac caaagggtta tgactgatga tgatctataa agggtgcaga 10320
   tgcaccctaa acatcaagag gggggaatga aatggagttt taactaaaaa acaatccccc 10380
   cccttggcta agaaggcagc tgcaagcctt tccagctttg ctcatcacta tgccaccctt 10440
   cttgtaataa tgtaacaact gtgtgccctc tgtccaagcc agcagccttg ctaaccaggt 10500
   acccagcatt gcttatcagt tccacttctg caaccttggt tgctcagttt cttggggagg 10560
   aacactgtct gcttggggat gggggaggtc cgggatgctt acatgggaaa atcaagacct 10620
   tgtagtgtat aaaagttact gagaacaaag acctggtgct cagactctag aggtgactct 10680
   ctgagcccgc accggtgctg aataaacctt gcttttccat atcttcgagt gctgtttgtc 10740
   tcctgttgcc acg 10753

## Description

The invention relates to the field of xenotransplantation and/or to the detection of retrovirus.

The number of patients requiring organ or tissue transplantation far outweighs the availability of suitable human donor organs. Xenotransplantation of non-human primate and pig transplants is therefore being considered as a means to alleviate this donor shortage. Although baboon-to-human xenotransplants have been attempted, most attention is currently being focused upon pigs as a suitable donor of cells, tissues and vascularized organs. Pig heart valve, skin, pancreas islet cell, and liver have indeed already been transplanted into patients. At present, these clinical successes have resulted in many more people desiring to be considered as candidate for xenotransplantation.

However, major problems have still to be overcome before xenotransplantation can be considered routine practice. Major obstacles are adverse immune reactions of the recipient against the xenotransplant; standing out among these adverse reactions is the hyperacute reaction (HAR) of xenotransplants, that result from the presence of xenoreactive antibodies to the alpha-Gal sugar moiety present on bacteria, which are also reactive with non-primate mamalian glycoproteins present on for example pig cells. The resulting complement activated immune reaction results in acute rejection of the transplant by the recipient.

Currently, however, specific xenograft sources such as transgenic pigs or pig populations are being produced that are genetically devoid of glycoproteins and other antigens comprising said alpha-Gal sugar moiety. If needed, further genetic modification will lead to pigs having also been modified in yet other adverse pig antigens. Genetic tailoring thus allows generating so-called "humanised" pigs and pig populations that can be used as graft source for cells, tissues or organs to be transplanted to man. Together with currently practiced immunosupressive techniques to help reduce adverse immune reactions even with common human-to-human transplantations, xenotransplantation with less adverse immunerecations will be possible.

However, yet another problem to overcome relates to the risk of infectious disease transmission from graft to recipient, and conceivably to further transmission among the host population. The normal DNA of mammals such as humans or pigs contains sequences related to infectious exogenous retroviruses which, even when they are not normally pathogenic in their natural host, are called endogenous retrovirus (ERV). Some of these ERVs, for example simian foamy virus, baboon retrovirus and pig endogenous retrovirus (PERV) A, B, and C (Takeuchi et al., J. Vir. 72:9986-9991, 1998) can or are thought to be capable to infect human cells, including human diploid fibroblasts, kidney, T- and B-cell lines. Therefore, the presence of ERV in the graft's genome creates a potential risk of zoonotic infection by retroviruses following xenotransplantation, especially when recipients are being immunosuppressed. Indeed, the FDA, for example, requires all porcine xenograft recipients in the USA to be monitored for porcine retrovirus infections.

If pigs are to be used as graft source for human xenotransplantation, it would also be advisable to eliminate or at least reduce the possibility of pig-to-human infection by retroviruses. Although pig retroviruses have not been directly shown to be pathogenic in pigs, some have been associated with lymphoma. Its possible replication in immunosupressed humans might lead to oncogenic or other undesirable events. Also, recombinatorial events of a porcine (be it exogenous or endogenous) retrovirus with for example a human endogenous retroviral sequence present in the recipient of a xenotransplant may render new viruses pathogenic in human hosts.

For example, the C-type virus most closely related to the generally known porcine retroviruses causes lymphoid and myeloid malignancies in gibbons. This gibbon ape leukemia virus spreads as an ape-to-ape infection among gibbons but appears to have recently evolved from retroviruses of South-East Asian mice. A replication-competent recombinant virus derived from a murine retroviral vector was also oncogenic in rhesus monkeys in which it replicated to high titre. Thus even if an endogenous retrovirus were non-pathogenic in its natural host there would be no room for complacency after transfer to humans (Patience et al., Nature Medicine 3:282-286, 1997; Stoye, Lancet 352:666-667, 1998). Similar cross-species events from chimpansees to humans are thought to have laid at the basis of the current HIV-epidemic.

Hence, to further evaluate or allow xenotransplantation, reliable screening methods for retroviral infections are needed, not only to determine the presence of, possibly as yet unknown, retroviral sequences in sources of xenotransplants or xenografts, but also to study graft recipients previously exposed to animals organs and cells, and last but not least to study those contact individuals closest to said recipients to determine the possible rate of transmission of non-human or recombinatory retroviral sequences to the human population at large.

The invention provides means and methods to determine exogenous or endogenous retroviral background in an animal, preferably a mammalian, such as a pig or primate, genome, including the determination of retroviral sequences, copy numbers and genome loci, and also provides clinical diagnostic kits to evaluate xenografts and monitor xenotransplant recipients and their contacts.

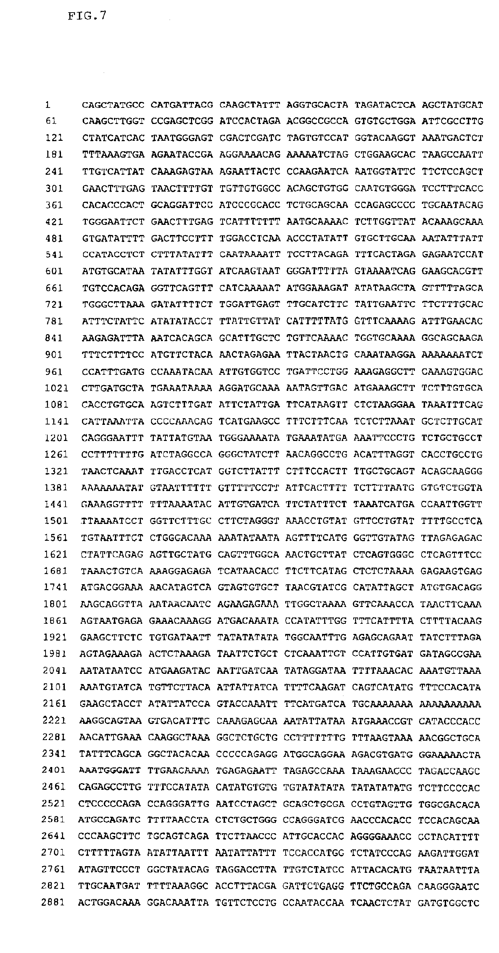

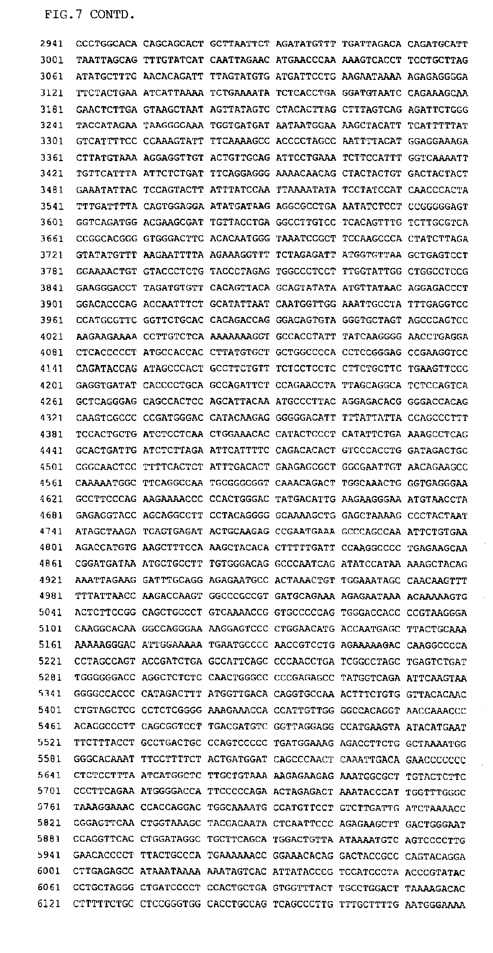

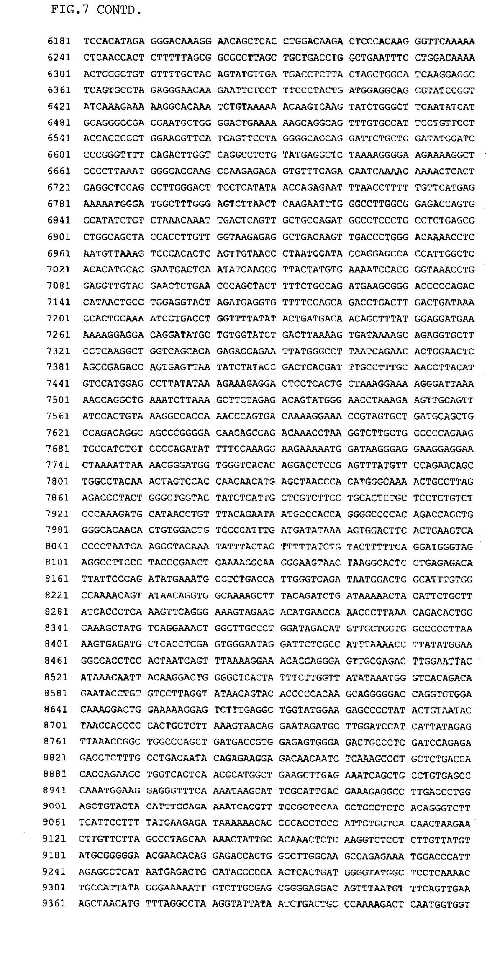

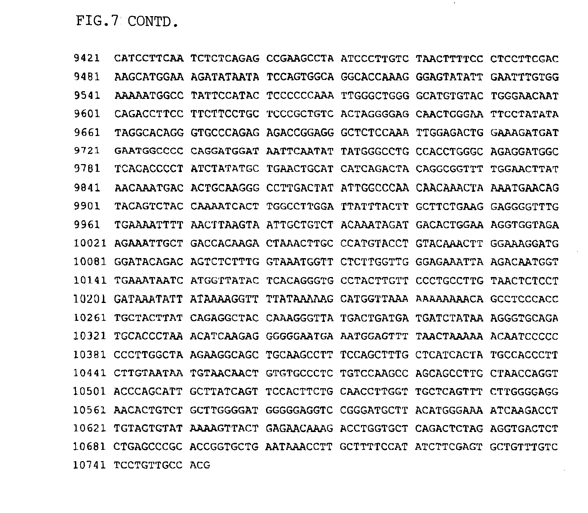

In a first embodiment, the invention provides a nucleic acid encoding or comprising a consensus retroviral gene sequence, a probe or a primer, allowing detection of a retroviral genome by nucleic acid hybridisation and/or amplification techniques known in the art, said consensus sequence at least partly derived from or hybridising with a domestic pig retrovirus genome comprising a sequence as shown in figure 7.

Said domestic pig retrovirus (DoPEV), as herein also provided, is only distantly related to preiviously known porcine enteroviruses, and even closer related to human endogonous enterovirus, thus bearing an even greater risk of recombination with a human virus when present in a xenotransplant to be given to a human.

In a preferred embodiment, and as further explained in the detailed description herein, the invention provides a nucleic acid wherein said sequence is also at least partly derived from or hybridising with a retroviral *pol* gene, said gene being one of the least complicated targets for detection. In another embodiment, the invention provides a nucleic acid wherein said sequence is also at least partly derived from baboon endogenous virus, giving also a more sensitive detection means (probe or primer) for detecting non-human primate ERV's.

In another embodiment, the invention provides an isolated or recombinant nucleic acid comprising at least a part of a nucleic acid derived from a domestic pig retrovirus genome comprising a sequence as shown in figure 7 or a sequence encoding an amino acid sequence at least 60%, preferably at least 70, more preferably at least 80%, most preferably at least 90% homologous to an amino acid sequence as encoded by a sequence as shown in figure 7. For example, at the amino acid level, the *gag, pol and* env genes of DoPEV have approximately 35, 55, and 45% homology to those genes of HERV (4-1), again illustrating the increased risk of recombination.

The invention also provides a method for detecting a retroviral genome or fragment thereof in a sample, such as a blood, tissue of cell sample derived from a mammal, such as a pig or baboon, comprising hybridising or amplifying at least a part of said genome, when present in said sample, with a probe or primer derived from a nucleic acid according to the invention. Such primers can for example be found in Table 2, but others are now easily designed using existing computer programmes, for example using sequence fragments derived from the *pol, gag* or *env* areas. In the detailed description such a method is described which comprises a polymerase chain reaction, however, other hybridisation (such as nucleic acid blotting, fluorescent *in situ* hybridisation) or amplification (such as NASBA) methods are well known in the art and herein included.

In particular, the invention provides use of a nucleic acid or a method according to the invention in testing of a mammalian xenotransplant source for the presence of a retroviral genome or fragment thereof. Such a source of course comprises an animal or animal population, preferably a transgenic or "humanised" animal, such as a pig, or an specific pathogen free (SPF) pig population, which are specifically produced or kept for xenotransplant purposes, or a graft derived from such an animal. However, the invention also provides such use in testing of a mammalian xenotransplant recipient or contact of said recipient for the presence of a retroviral genome or fragment thereof.

Also, the invention provides a natural (monoclonal or polyclonal) or synthetic (e.g. phage library expressed or obtained) antibody specifically direct against a protein or fragment thereof derived from a domestic pig retrovirus genome comprising a sequence as shown in figure 7 or a sequence homologous thereto, as provided herein. Such antibodies are now, having an antigen source available, easily made, and the invention thus also provide use of such an antibody in testing a sample for the presence of a retroviral fragment, for example in a method for determining the presence of retroviral fragments in a sample comprising immunological detection of a retroviral fragment with an antibody such as with an ELISA test.

Distinct nucleic acid sequences, probes, primers, or antibodies can easily been made parts of a diagnostic kit, which is herewith provided, said kit optionally further comprising means that are further desired to execute or perform a method according to the invention. Such kits, probes, primers or antibodies are useful in a method for testing a mammalian xenotransplant source, recipient or contact of said recipient for the presence of a retroviral genome or fragment thereof comprising testing a sample obtained from said source. The invention thus provides a graft, obtainable with a method according to the invention, comprising a mammalian cell, tissue or organ for use in xenotransplantation having been tested for the presence of a retroviral genome or fragment thereof. In particular, the invention provides such a graft comprising a porcine cell, tissue or organ, preferably at least tested for the presence of DoPEV, or viruses related thereto, allowing a improved method for the treatment of an individual having been diagnosed with a failure of cells, tissue or organ comprising providing said individual with a graft tested according to the invention.

The invention is further described in the detailed description without limiting the invention thereto.

### Figure legends

Fig.1: PCR amplification of pig retroviral RT gene by BBRT primers. Lane 1- 4: serial dilution of pig genomic DNA, from 10⁻¹ to 10⁻⁴; M: 100 bp marker. The target fragment is shown by an arrow.

Fig.2: PCR amplification of pig retroviral RT gene by LH primers. Lane 1- 4: serial dilution of pig genomic DNA, from 10⁻¹ to 10⁻⁴; M: 100 bp marker. Lane 5: Amplification of BaEV RT as a positive control. The target fragment is shown by an arrow. M: DNA molecular marker.

Fig. 3 Sequence alignment of 5 RT sequences amplified from pig genome by BBRT and LH primer sets. A consensus sequence is shown at bottom line of alignment.

Fig.4 Gene map of 11 kb sequence identified from P1.1 and motifs in the retroviral genes

Fig. 5. NJ phylogenetic tree based on the *gag* amino acid sequences for DoPEV, HERV(4-1), and other C-type retroviruses. P11: DoPEV; PK15: porcine endogenous retrovirus (PERV) ; GaLV : gibbon ape leukaemia virus ; MuLV : murine leukemia virus ; BaEV : baboon endogenous retrovirus, showing that the DoPEV is most closely related to the human endogenous virus.

Fig. 6. NJ phylogenetic tree based on the *gag* amino acid sequences for DoPEV, HERV(4-1), and other C-type retroviruses. P11: DoPEV; PK-15: porcine endogenous retrovirus (PERV) ; GaLV : gibbon ape leukemia virus ; MuLV : murine leukemia virus ; BaEV : baboon endogenous retrovirus, again showing that the DoPEV is most closely related to the human endogenous virus.

Fig. 7. Nucleic acid sequence of DoPEV genomic fragment comprising a 11 kb sequence obtained from clone P1.1.

### Detailed description.

### Materials, methods and results

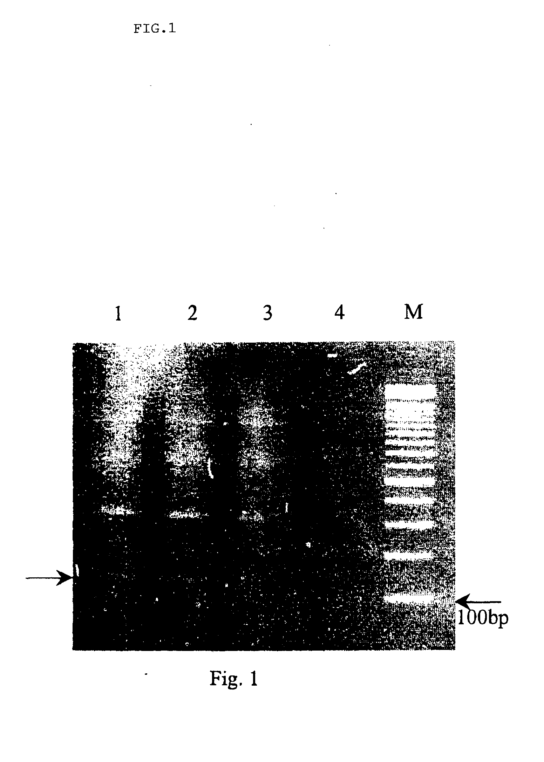

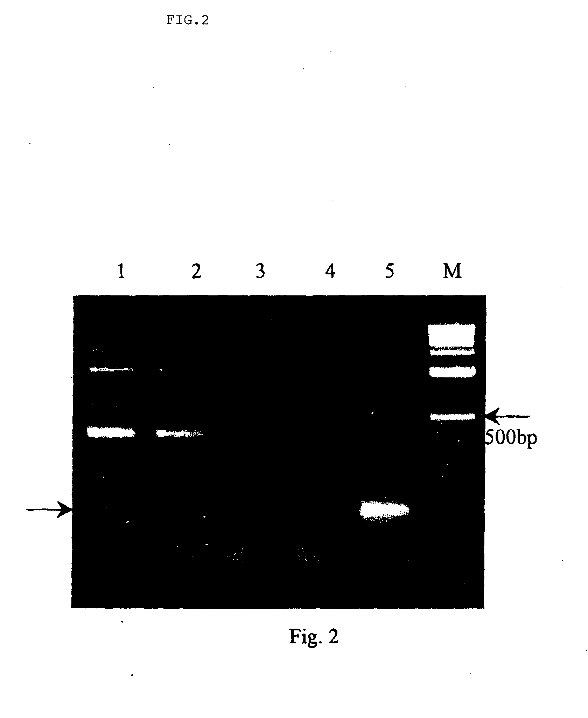

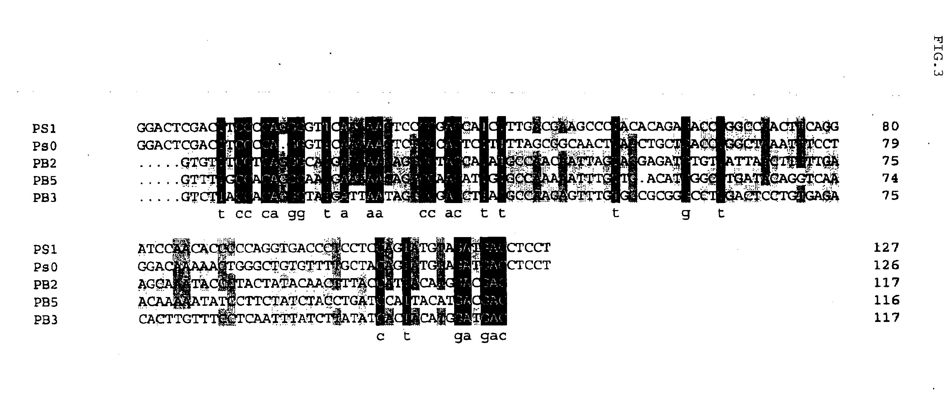

Identify different types of RT sequences from pig genome by PCR
1. Two sets of primer were designed to amplify the core fragment of retroviral RT gene
   1) Degenerated BBRT set was designed based on a consensus retroviral RT sequence:
      - BBRT1: 5'- CTCGGATCCGTNYTNCCNCARGG -3' (upstream)
      - BBRT2: 5'- CTCGTCGACRTCRTCCATRTA -3' (downstream)
      With this primer set, a 130 nt retroviral RT fragment could be amplified.
   2) LH set was designed based on the RT sequence of baboon endogenous virus (BaEV), which has a C type *pol* gene:
      - L3505: 5'- TGGACTCGACTTCCCCAGGG -3' (upstream)
      - H3616: 5'- TATAGCGGCCGCAGGAGGTCATCTACATA -3'(downstream)
      With this primer set, a type C retroviral RT fragment which might be approximately 130 nt in length could be amplified.
2. Prepare pig genomic DNA
   1) 10⁷ pig PBMC was isolated from whole blood, and suspended in 1 ml RPM 1640 medium.
   2) The silica based Y isolation was performed with 10⁶ PBMC, and total nucleic acid was dissolved in 50 µl distilled H20.
3. PCR amplification of retroviral RT core fragment
   1) Pig genomic DNA was diluted serially by distilled H20 from 10⁻¹ to 10⁻⁴ (v/v), and PCR amplifications were performed with BBRT primers and LH primers respectively. A plasmid which contains the RT gene of BaEV was used as positive control.
   2) The program for the BBRT and LH PCR reactions are: 94°C hold for 5 minutes; 94°C 1 minute, 37°C 2 minutes, 72°C 3 minutes for 10 cycles; 94°C 30 seconds, 55°C 1 minute, 72°C 1 minute for 30 cycles, then 72°C hold for 10 minutes. PCR results are shown in Fig. 1 and Fig. 2.
4. The target fragments of PCR amplification were recovered from agarose gel by QIAEX II kit (Qiagen), cloned into pCRII-TOPO vector (Invitrogen) and sequenced with an Applied Biosystems 373A automated sequencer with T7 dye primer by following manufacturer's protocols. 5 different RT sequences were identified by gene sequencing, and they were named PB2, PB3, PB5 and PS1. PB2, 3, and 5 were obtained by BBRT amplification, and the nucleotide homology among them is higher than the homology to PSO and PS1 which were obtained by LH amplification. PSO and PS1 are very homologous each other, as shown in Fig. 3. For the homology of these 5 sequences and other retroviral RT core sequences, PB2, 3 and PB5 are very homologous to the core fragment of D-type retroviruses, including SMRV; PSO is very homologous to some C-type retroviruses, like BaEV and MuLV; and PS1 is almost identical to the RT core fragment of PERV (99% homology).

### Screen pig genomic DNA library with RT probes

1. A pig genomic DNA library was obtained from Stratagene. 5 different RT probes were equally mixed and labelled by (α-³²P) dCTP. About 60,000 λ phage plaques were screened by the Southern blot with the probe mixture for two rounds. 26 positive clones were identified.
2. Some of these 26 clones may contain the viral genome of previously known PERV. In order to reduce the background of PERV, 2 primers were synthesised based on the sequence of PERV protease gene, and a 320 nt amplification target fragment was expected.
   - PERV-FP: 5'- GCTACAACCATTAGGAAAACTAAAAG -3' (upstream)
   - PERV-RP: 5'- AACCAGGACTGTATATCTTGATCAG -3' (downstream)
   10 of 26 clones were determined to contain the genome of PERV by PCR with this primer set.
3.16 λ clones containing non-PERV retroviral sequence were left after all screening procedures. The insert of one clone, named P1.1 was sequenced by GAT system.

### Gene sequencing of P1.1 insert fragment with GAT system

1. λ DNA of P1.1 was isolated by using the Wizard Lambda Preps DNA purification system (Promega). The insert fragment was separated from two arms by Not I digestion and agarose electrophoresis and purified by using the Quantum Prep Freeze `N Squeeze Spin Column (Bio-rad). The length of P1.1 insert is about 23 kb.
2. The purified P1.1 insert fragment was randomly amplified by the GAT system. More than 100 subclones were generated and sequenced from 5' and 3' directions respectively by an Applied Biosystems 377 automated sequencer with Big-dye Terminator cycle sequencing kit (Perkin-Elmer Applied Biosystem).
3. Sequencing data was analysed by the Auto Assembler DNA sequence assembly software (Perkin-Elmer Applied Biosystem). A 11 kb sequence which contains retroviral-like sequences was obtained after assembling and the sequence was checked and corrected manually.

### Sequence analysis of P1.1

Result of sequence alignment showed that P1.1 contains at least a partial genome of a novel C type porcine endogenous retrovirus, which we named domestic pig endogenous retrovirus (DoPEV) .
To gain more information about the viral genome in P1.1, we blasted the llkb nucleotide sequence and the amino acid sequences translated from all three ORFs to GeneBank to search the homology between P1.1 containing genes and other genes, especially for C-type viruses and pig genes. Results of blasting are summarised in table 1.
The results suggested that P1.1 comprises fragments or parts of the genome of a novel porcine endogenous retrovirus, DoPEV, including *gag, pol* and *env* genes and 3' LTR.

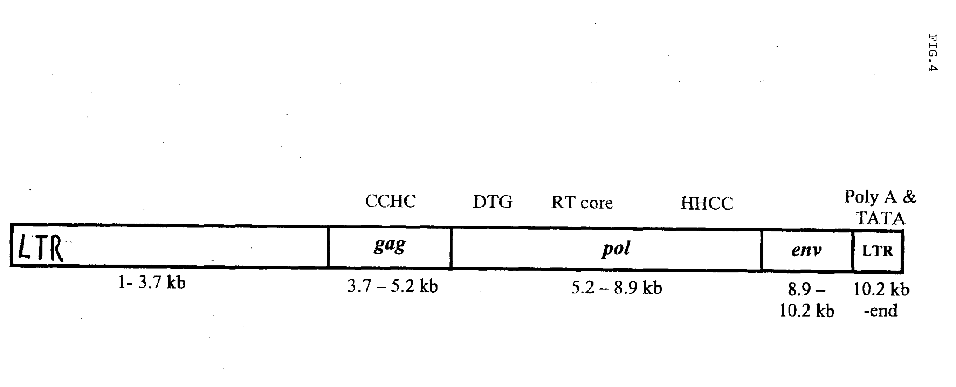

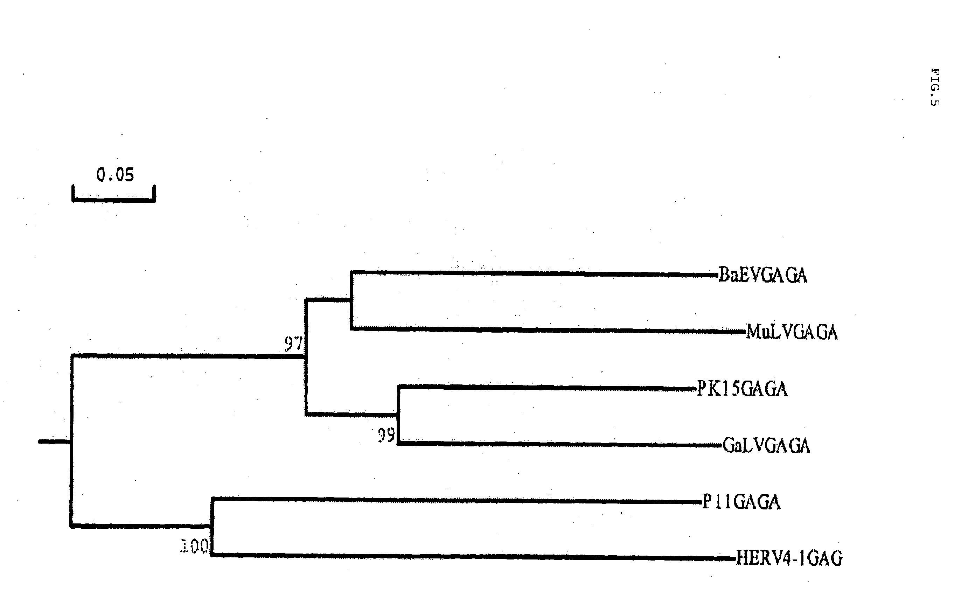

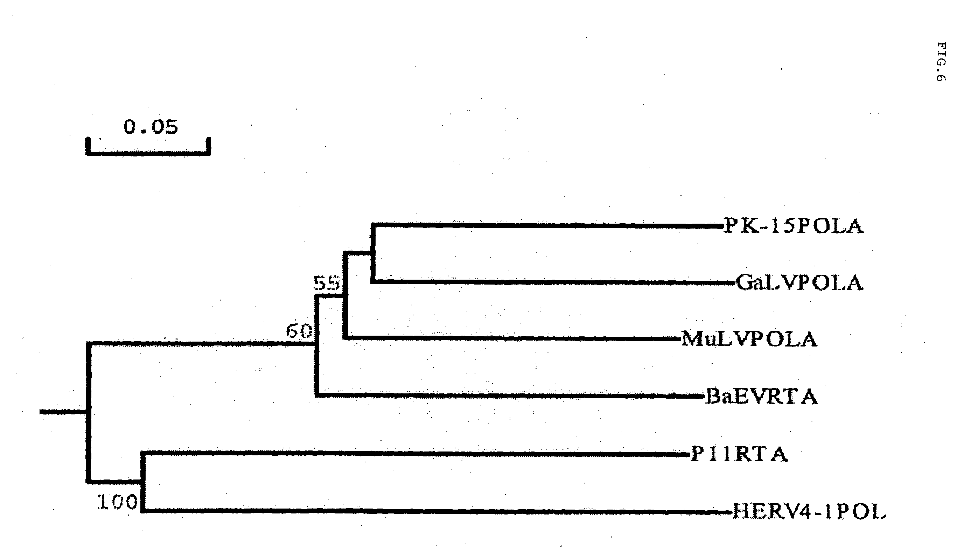

2. To confirm our prediction, several retroviral nucleotide and amino acid sequence motifs were searched along P1.1 containing sequence and almost all important motifs could be found, including (see also figure 4):
   - In the Gag region: CCHC motif, which is in the p10 polypeptide region and required for efficient viral RNA packing
   - In the Pol region: 1) DTG motif, which is highly conserved among the proteases of all retroviruses; 2) VPLQG...YMDD motif in the RT core region; 3) HHCC motif, which is required for retroviral endonuclease function.
   - In the LTR region: the polyadenylation (Poly A) site and the TATA box
3. To determine the homology between DoPEV and other C-type retroviruses, amino acid sequence alignment and phylogenetic analysis based on *gag, pol,* and *env* genes were performed (Fig. 5 and 6).
   Sequence alignment and phylogenetic analysis results of *gag, pol* and *env* genes of DoPEV, HERV (4-1) and other C-type retroviruses showed that the most homologous retrovirus to DoPEV is HERV (4-1), which is a C-type human endogenous retrovirus with about 35 to 50 copies through human genome. At amino acid level, the *gag, pol and* env genes of DoPEV have more than 35, 55, and 45% homology to those genes of HERV (4-1) .
4. In order to determine if any other clone we obtained from pig library screening also contains DoPEV genome, 16 primers were synthesised based on the P1.1 llkb sequences, and PCR was performed by using λ DNA as template. The primer sequences and PCR results are shown in tables 2 and 3, respectively.
   The results of PCR amplification for the λ DNA with PC serial primers suggested 12 λ clones including P1.1 which we screened from pig genomic library contain at least partial genome of DoPEV. Some clones, for an example P14.1 and P26.1, probably contain the full genome of DoPEV. As the patterns of PCR result are different form clone to clone, it shows that multiple copies of DoPEV are present in the pig genome.

### Determining DoPEV expression in pig PBMC by RT-PCR

1. To determine if part of the *gag* and *pol* genes of DoPEV are expressing in pig PBMC, other two primers sets were designed based on the sequence we obtained from P1.1
- Gag primer set: 5'- CGATGGGACCATACAAGAGG -3' (PC41, upstream)
   5'- GCCAAGTCTGTTTGACCC -3' (PC4, downstream)
- Pol primer set: 5'- CCTAATGGATACCAGGAGCC -3' (PCRT1, upstream)
   5'- CAGTCAAGTCAGGTCTGCTGG -3' (PC10, downstream)
   3) For each RT-PCR reaction, RNA isolated from 10⁶ pig PBMC and OligodT(16) were used as template and the 3' primer for reverse transcription. RT product was amplified with the Gag, Pol, BBRT, LH, primer sets. The amplification condition is: 94°C hold for 5 minutes; 94°C 1 minute, 37° C 2 minutes, 72°C 3 minutes for 10 cycles; 94°C 30 seconds, 55°C 1 minute, 72°C 1 minute for 30 cycles, then 72°C hold for 10 minutes.

RT-PCR results showed that the DoPEV *gag* and RT genes are expressing in pig cells.

The partial genome of a novel porcine endogenous retrovirus, DoPEV, is identified from a λ phage clone, which is obtained from a pig genomic DNA library by the Southern blot with probes containing the RT core fragments of C type retrovirus. Gene sequences of DoPEV *gag, pol,* and *env* genes and 3'LTR are obtained. Multiple copies of DoPEV are present in the pig genome. The *gag* and RT genes of DoPEV are expressing in pig PBMC cells.

**1. Table 1**

| Sequence Region(nt) | Most Homologous Gene | Percentage of homology (AA1/NT2) | Remarks |
|---|---|---|---|
| 1 - 114 | Some cloning vectors | > 95 (NT) | 5' flanking sequence of P1.1 insert |
| 214 - 2500 | No homology to any known pig and virus gene | | Probably is the unknown pig gene |
| 2525 - 2800 | Sus *scrofa* MHC class I SLA gene and proteolipid protein gene | > 90 (NT) | Viral flanking sequence |
| 2801 - 3700 | No homology to any known pig and virus gene | | |
| 3740 - 5200 | The gag gene of many C type viruses, including HERV (4-1)³ | ~ 35 (AA) | Containing the full length gag gene for DoPEV |
| 5300 - 8900 | The *pol* gene of many C type viruses, including HERV (4-1)³ | ~ 55 (AA) | Containing the full length *pol* gene for DoPEV |
| 8940 - 10240 | The env gene of HERV (4-1)³ | ~ 40 (AA) | Containing the partial env gene for DoPEV |
| 10241-end | No homology to any known pig and virus gene | | Probably containing the 3' LTR of DoPEV |

| | | | |
|---|---|---|---|
| ¹AA: amino acid level homology; ²NT: nucleotide level homology; ³HERV (4-1): human endogenous retroviral DNA (4-1), accession number: M10976 | | | |

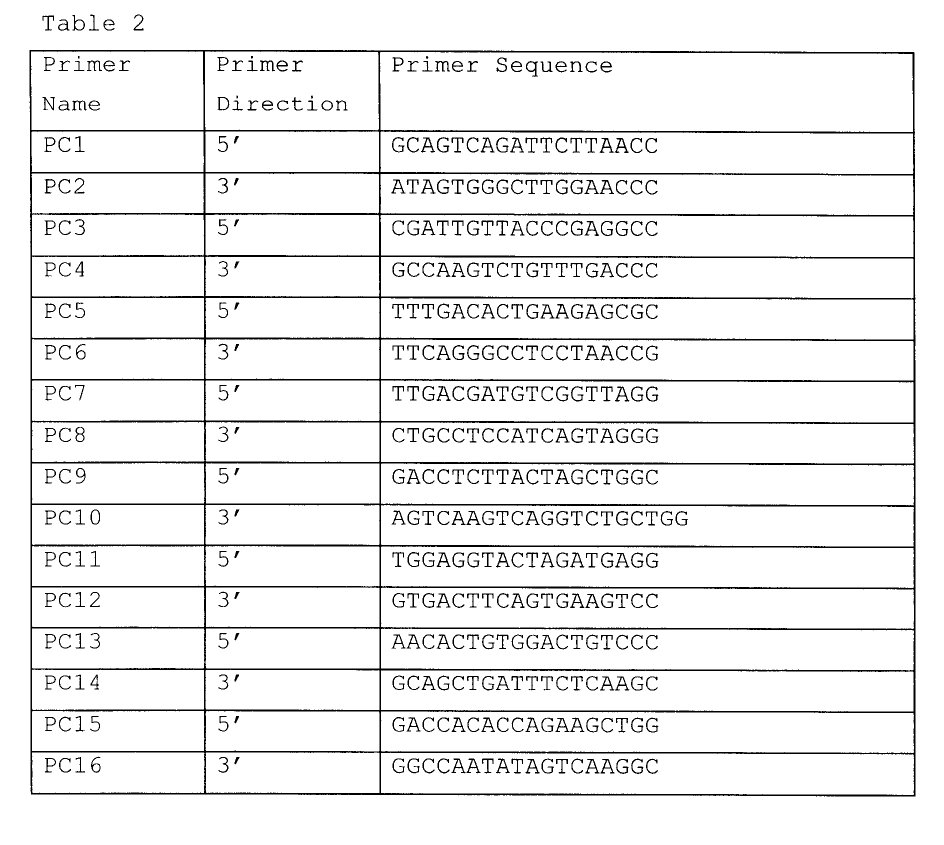

**Table 3**

| λ ^{*clones*} | *PC1-2* | *PC3-4* | *PC5-6* | *PC7-8* | *PC9-10* | *PC11-12* | *PC13-14* | *PC15-16* |
|---|---|---|---|---|---|---|---|---|
| P1.1 | 0.9kb | 0.9kb | 0.9kb | 0.9kb | 0.9kb | 0.9kb | 0.9kb | 0.9kb |
| P2.1 | - | 0.9kb | 0.9kb | 0.9kb | - | - | - | 2.0kb |
| P3.1 | 2.0kb | - | 1.8kb | - | - | - | - | - |
| P4.1 | - | - | - | - | - | - | 0.9kb | 0.9kb |
| P9.1 | - | 1.1kb | 1.1kb | 0.9kb | 0.9kb | - | - | - |
| P12.1 | - | - | 1.8kb | 0.9kb | - | - | - | - |
| P14.1 | - | 0.9kb | 0.9kb | 0.9kb | 0.9kb | 0.9kb | - | 0.9kb |
| P19.1 | - | 0.9kb | 1.8kb | - | 0.9kb | 0.9kb | 0.9kb | 1.5kb |
| P23.1 | 1.4kb | 0.9kb | 0.9kb | 0.9kb | - | - | - | - |
| P26.1 | - | - | 0.9kb | 1.1kb | 0.9kb | 0.9kb | 0.9kb | 0.9kb |
| P27.1 | - | - | 1.8kb | - | - | - | - | - |
| P28.1 | - | - | 1.8kb | - | - | - | 0.9kb | - |
| *The lengths of specific PCR product are shown in the table. -: No specific PCR products could be determined. | | | | | | | | |

## Claims

1. An isolated or recombinant nucleic acid comprising a consensus retroviral sequence allowing detection of a retroviral genome by nucleic acid hybridisation and/or amplification, said consensus sequence at least partly derived from a domestic pig retrovirus genome comprising a sequence as shown in figure 7.

2. A nucleic acid according to claim 1 wherein said sequence is also at least partly derived from or hybridising with a retroviral *pol* gene.

3. A nucleic acid according to claim 1 or 2 wherein said sequence is also at least partly derived from baboon endogenous virus.

4. An isolated or recombinant nucleic acid comprising at least a part of a nucleic acid derived from a domestic pig retrovirus genome comprising a sequence as shown in figure 7 or a sequence encoding an amino acid sequence at least 60% homologous to an amino acid sequence as encoded by a sequence as shown in figure 7.

5. A method for detecting a retroviral genome or fragment thereof in a sample comprising hybridising or amplifying at least a part of said genome with a probe or primer derived from a nucleic acid according to anyone of claims 1 to 4.

6. A method according to claim 5 which comprises a polymerase chain reaction.

7. Use of a nucleic acid according to anyone of claims 1 to 4 or a method according to claim 5 or 6 in testing of a mammalian xenotransplant source for the presence of a retroviral genome or fragment thereof.

8. Use according to claim 7 wherein said xenotransplant source comprises a cell, tissue or organ.

9. Use according to claim 7 or 8 wherein said xenotransplant source comprises a pig.

10. Use of a nucleic acid according to anyone of claims 1 to 4 or a method according to claim 5 or 6 in testing of a mammalian xenotransplant recipient or contact of said recipient for the presence of a retroviral genome or fragment thereof.

11. An antibody specifically direct against a protein or fragment thereof derived from a domestic pig retrovirus genome comprising a sequence as shown in figure 7 or a sequence at least 60% homologous to a sequence as shown in figure 7.

12. Use of an antibody according to claim 11 in testing a sample for the presence of a retroviral fragment.

13. A method for determining the presence of retroviral fragments in a sample comprising immunological detection of a retroviral fragment with an antibody according to claim 11.

14. A diagnostic kit comprising a nucleic acid according to anyone of claims 1 to 4 or an antibody according to claim 12.

15. A method for testing a mammalian xenotransplant source, recipient or contact of said recipient for the presence of a retroviral genome or fragment thereof comprising testing a sample obtained from said source with a method according to claim 5, 6 or 12.

16. A graft comprising a mammalian cell, tissue or organ for use in xenotransplantation having been tested for the presence of a retroviral genome or fragment thereof with a method according to claim 5 or 6.

17. A graft comprising a porcine cell, tissue or organ according to claim 16.

18. A method for the treatment of an individual having been diagnosed with a failure of cells, tissue or organ comprising providing said individual with a graft according to claim 16 or 17.
